# EUROPEAN PATENT APPLICATION

(11) **EP 4 650 777 A1**
(43) Date of publication of application: **19.11.2025**
(21) Application number: 24382518.9
(22) Date of filing: 13.05.2024
(51) Int. Cl.: G01N 33/569

(54) **NOVEL METHOD AND FLOW CYTOMETRY PANELS FOR IMMUNOPHENOTYPING**

(71) Applicant: Fundación para la Investigación Biomédica del Hospital Gregorio Marañón (FIBHGM), 28007 Madrid (ES); Universidad Carlos III de Madrid (UC3M), 28911 Leganés (Madrid) (ES)
(72) Inventor: BARCO TEJADA, Ainara, 28911 Leganés (ES); LÓPEZ ESTEBAN, Rocío, 28007 Madrid (ES); BLÁZQUEZ LÓPEZ, Elena, 28007 Madrid (ES); DESCO MENÉNDEZ, Manuel, 28911 Leganés (ES); CUSSÓ MULA, Lorena, 28007 Madrid (ES); CORREA ROCHA, Rafael, 28007 Madrid (ES); PION, Marjorie, 28007 Madrid (ES)
(74) Representative: Hoffmann Eitle

(57) **Abstract**

The present invention relates to a novel method for flow cytometric immunophenotyping of a mouse from a very small volume of blood using a novel combination of markers. The invention furthermore relates to a reagent composition for flow cytometric immunophenotyping of blood cells comprising fluorochrome-conjugated antibodies directed against a combination of markers.

## Description

### TECHNICAL FIELD

The present invention relates to a novel method for flow cytometric immunophenotyping of a mouse from a very small volume of blood using a novel combination of markers. The invention furthermore relates to a reagent composition for flow cytometric immunophenotyping of blood cells comprising fluorochrome-conjugated antibodies directed against a combination of markers.

### BACKGROUND OF THE INVENTION

Flow cytometry (FCM) is a fluorescence-based technique that allows studying the optical properties of cells as well as quantifying them when they are suspended in a flow, enabling the simultaneous study of numerous cellular markers (1,2). Therefore, FCM is a fundamental tool in the diagnosis and progression of immunological conditions such as HIV immunodeficiencies and oncological diseases (3-5), among others. In addition to its clinical applications, FCM is widely used in preclinical research (2). Specifically, mice are the most commonly used animal species in research (6). However, despite presenting immune markers that are equal or similar to humans, the application of FCM in mice presents several limitations that hinder the design of cytometry panels (7-9): 1) The limited availability of mouse antibodies compared to the variety of fluorochromes available in conventional cytometers; 2) the need to work with small volumes of peripheral blood (PB) (10), which particularly complicates the conduct of longitudinal studies since serial extractions cannot exceed 10% of the mouse's blood volume (10, 11); and 3) in these volumes, the quantity of immune cells is small, so minority populations exhibit greater variability and may thus be more influenced by external factors such as anesthesia, PB collection method, or the volume of PB extracted (12).

Therefore, most FCM studies in mouse (13,14) are conducted on isolated organs (15), mainly the spleen, due to its ease of extraction and the abundance of immune cells it contains (10). This means that the few studies on innate and adaptive immune populations in PB have been conducted with simple panels or focused on specific populations (13, 14, 16).

There is therefore a need to provide for improved methods for diagnosis and longitudinal monitoring of progression of immunological conditions in the mouse model that overcome the above disadvantages. The inventors have therefore set out to develop an improved method that allows extensive analysis of immune cell populations in mice in longitudinal studies and that can serve as a valuable tool in clinical research.

### SUMMARY OF THE INVENTION

The present invention therefore in one aspect relates to a method for immunophenotyping of a mouse comprising the steps of:
(i) providing a blood sample of the mouse,
(ii) selecting a panel of fluorescently labeled antibodies,
(iii) incubating the selected panel of fluorescently labeled antibodies with the blood sample to be tested,
(iv) detecting the signal by flow cytometry to obtain detection data, and
(v) determining the cell type according to the data obtained in step (iv),

wherein the panel of fluorescently labeled antibodies comprises at least one of the following sets of markers:
   a) CD45, Ly6C, B220, CCR2, CD11b, CD11c, CD172a, CD49b, CD80, CD86, F4/80, Ly6G, MHC-II, Siglec-F, or
   b) CD45, CCR4, CCR6, CD138, CD19, CD25, CD3, CD4, CD44, CD62L, CD8, CXCR3, TNF-RII, NK1.1, and
wherein the volume of the blood sample of the mouse provided in (i) is less than 100 µl per set of markers.

In one embodiment of the method of present invention the selected panel comprises both sets of markers (a) and (b).

In one embodiment of present invention the blood sample of the mouse provided in step (i) is less than about 80 µl, preferably less than about 70 µl, most preferred less than about 60 µl per set of markers.

In one embodiment of present invention the blood sample of the mouse provided in step (i) is between about 50 to 100 µl per set of markers, most preferred is an amount of between about 50 to 80 µl per set of markers.

In one embodiment the blood sample is a peripheral blood (PB) sample.

In one embodiment of the method of present invention the step (v) of determining the cell type according to the data obtained in step (iv) comprises simultaneously identifying more than 30, preferably more than 40, most preferred more than 50 subpopulations of cells.

In one embodiment of the method of present invention the step (v) of determining the cell type according to the data obtained in step (iv) comprises obtaining the total amount of cells of each subpopulation as an absolute value.

In a second aspect the present invention relates to a reagent composition for flow cytometric immunophenotyping of blood cells comprising fluorochrome-conjugated antibodies directed against one of the following combination of markers:
a) CD45, Ly6C, B220, CCR2, CD11b, CD11c, CD172a, CD49b, CD80, CD86, F4/80, Ly6G, MHC-II, Siglec-F; or
b) CD45, CCR4, CCR6, CD138, CD19, CD25, CD3, CD4, CD44, CD62L, CD8, CXCR3, TNF-RII, NK1.1.

In one embodiment of this aspect the blood cells are peripheral blood (PB) cells.

In one embodiment of the reagent composition or the method of present invention each antibody in the combination of markers (a) and (b) is conjugated to one of the following fluorochromes: VioBlue, VioGreen, BV570, BV605, BV650, FITC, PE, ECD/PE-Vio615, PerCP/PE-Cy5/PC5, PerCP-Vio700/PC5.5, PE-Cy7/PE-Vio700/PC7, APC, AF700/APC5, APC-Fire^{™} 750/APC7.

In a preferred embodiment each antibody in the combination of markers (a) and (b) is conjugated to a different fluorochrome.

### BRIEF DESCRIPTION OF THE FIGURE

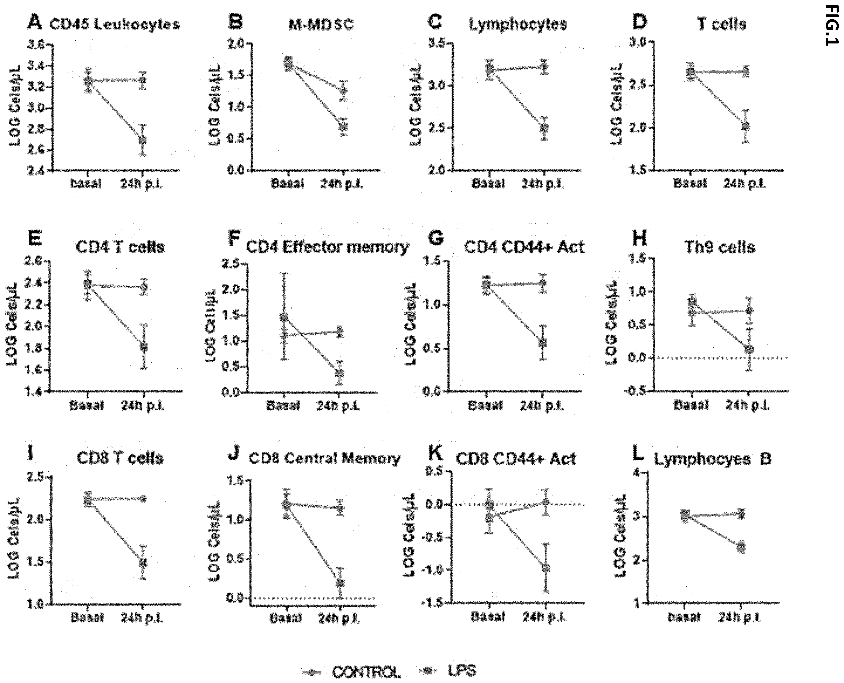

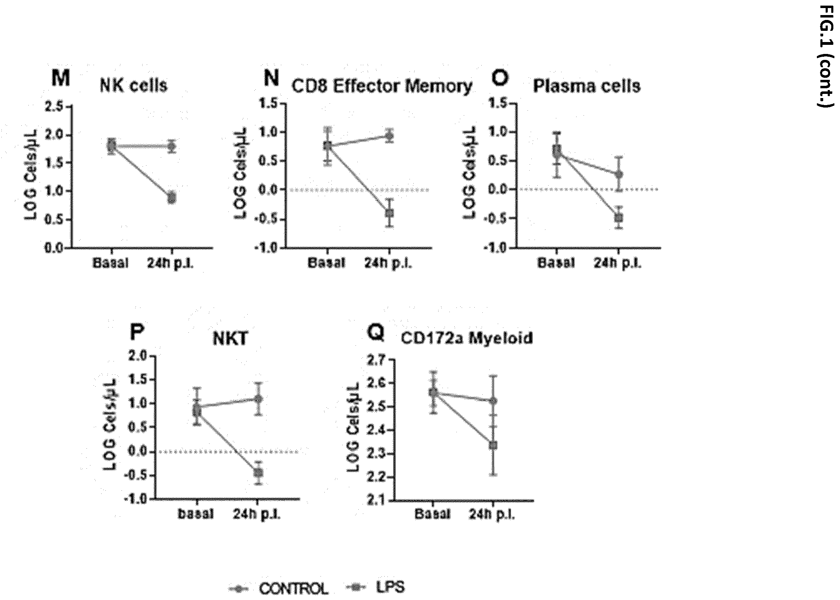

Figure 1: Variation at 24h p.i. of myeloid and lymphoid population of LPS and control animals expressed in absolute numbers. Only populations with significant differences between groups are shown (A-M) ***p≤0.001; (N, O) **p≤0.01; (P, Q) *p≤0.05.

### DETAILED DESCRIPTION

The present invention may be understood more readily by reference to the following detailed description of the preferred embodiments of the invention, and to the example included therein.

As described in more detail above, there is a need to provide for improved methods for diagnosis and monitoring of progression of immunological conditions in the mouse model that overcome the disadvantages of the currently available methods. The inventors have therefore set out to develop an improved method that allows extensive analysis of immune cell populations in mice in longitudinal studies and that can serve as valuable tools in clinical research.

To overcome the existing limitations, the inventors have defined novel cytometry panels suitable for analysing immune cell populations in mice in a longitudinal study from a very small sample of as little as 50µl of peripheral blood. The novel cytometry panels have been validated in a lipopolysaccharide (LPS) lung inflammation model as explained in more detail below.

The designed panels allow characterizing a high number of immune subpopulations in mice from a very small blood volume. This facilitates the conduct of longitudinal studies without interfering with the animal's physiology. The panels of present invention comprising 14 markers each overcome many of the limitations previously encountered in FCM studies of peripheral blood in mouse when using very small blood volumes. To date no methodologies using such a small blood volume have been described in the field.

For example, Breznik and colleagues extracted blood from the retro-orbital sinus, without animal recovery due to the need for larger volumes (18). Additionally, most research analyses PB cytometry only at the end point of the experiment, where all circulating blood from the animal (≈500 µL) is collected to be used in various FCM panels or other techniques such as immunohistochemistry (19-21).

The present invention therefore in one aspect relates to a method for immunophenotyping of a mouse, preferably a mouse, comprising the steps of:
(i) providing a blood sample of the mouse,
(ii) selecting a panel of fluorescently labeled antibodies,
(iii) incubating the selected panel of fluorescently labeled antibodies with the blood sample to be tested,
(iv) detecting the signal by flow cytometry to obtain detection data, and
(v) determining the cell type according to the data obtained in step (iv),

wherein the panel of fluorescently labeled antibodies comprises at least one of the following combination of markers:
   (a) CD45, Ly6C, B220, CCR2, CD11b, CD11c, CD172a, CD49b, CD80, CD86, F4/80, Ly6G, MHC-II, Siglec-F; or
   (b) CD45, CCR4, CCR6, CD138, CD19, CD25, CD3, CD4, CD44, CD62L, CD8, CXCR3, TNF-RII, NK1.1; and
wherein the volume of the blood sample of the mouse provided in (i) is less than 100 µl per set of markers.

In one embodiment the panel of fluorescently labeled antibodies consists of the following combination of markers:
(a) CD45, Ly6C, B220, CCR2, CD11b, CD11c, CD172a, CD49b, CD80, CD86, F4/80, Ly6G, MHC-II, Siglec-F.

In one embodiment the panel of fluorescently labeled antibodies consists of the following combination of markers:
(b) CD45, CCR4, CCR6, CD138, CD19, CD25, CD3, CD4, CD44, CD62L, CD8, CXCR3, TNF-RII, NK1.1.

In one embodiment the panel consists of both combinations of markers (a) and (b).

In one embodiment of present invention the blood sample of the mouse provided in step (i) is less than about 80 µl, less than about 70 µl, less than about 60 µl, less than about 55 µl per set of markers. The volume of the blood sample indicated herein is used per set of markers used in the panel, i.e., if the panel consist of the combination of markers (a) or (b) then the amounts indicated are used. If the panel consists of both combination of markers ((a) and (b)), then the amount of blood sample needed is doubled. In a preferred embodiment in which both combinations of markers are assessed about 100 µl sample are used (about 50 µl for each one).

In one embodiment the blood sample of the mouse provided in step (i) is between about 50 to 100 µl, between about 50 to 90 µl, between about 50 to 80 µl, between about 50 to 70 µl, between about 50 to 60 µl.

As explained above, the extremely small sample volume compared to the sample volumes used in the prior art allows for better handling of the mouse. The extraction of smaller amounts of peripheral blood poses less stress on the mouse and makes it possible to keep the animal alive and healthy as opposed to the methods of the prior art in which the animal is sacrificed for extracting sufficient amount of sample, or even extracting whole organs for the analysis.

A further important advantage of the method and panels of present invention is the high number of subpopulations that can be assessed with the panels thanks to the careful selection of the markers used. The panels of present invention allow the study of more than 60 subpopulations of immune cells whereas in previous studies the number of study populations is significantly reduced by using fewer markers (20, 22) or equipment with fewer fluorescence channels (21).

Up to now, despite the complexity of the immune response, which involves multiple interrelated lineages, most FCM studies in PB have only focused on major populations (23), such as total lymphocytes (24), or specific immunological parameters, such as cytokines (25). Even when working with isolated organs in FCM studies, the number of study populations is very limited (26, 27, 28).

Therefore, in one embodiment of the method of present invention the selected panel of fluorescently labeled antibodies comprises one of the following sets of antibodies:
(a) CD45, Ly6C, B220, CCR2, CD11b, CD11c, CD172a, CD49b, CD80, CD86, F4/80, Ly6G, MHC-II, Siglec-F, or
(b) CD45, CCR4, CCR6, CD138, CD19, CD25, CD3, CD4, CD44, CD62L, CD8, CXCR3, TNF-RII, NK1.1.

In one embodiment of the method of present invention the selected panel comprises both sets of antibodies (a) and (b).

Each set ((a) and (b)) contains 14 antibodies. Set a) has been specifically selected to detect myeloid cells and set b) has been specifically selected to detect lymphoid cells. Both panels are therefore highly specific. The careful selection of the antibodies in each of these panels has been made from a large number of possible antibodies. Specifically, the selection of the 14 antibodies of (a) allows the detection of a surprisingly large number of subpopulations from myeloid cells from a very small blood sample. Equally, the selection of the 14 antibodies of (b) allows the detection of a surprisingly large number of subpopulations from lymphoid cells from a very small blood sample. As explained above, the great benefit of the use of such small sample volumes is that it allows the mouse model to be kept alive and healthy during and after sample taking.

In one embodiment of the method of present invention the step (v) of determining the cell type according to the data obtained in step (iv) comprises simultaneously detecting more than 30, more than 35, more than 40, more than 45, more than 50, more than 55, subpopulations of cells.

The subpopulations of cells for immunophenotying are known in the prior art and not limited to the ones disclosed herein. The subpopulations detected with the panels of present invention are shown in table 3 and 4, but it is to be understood that further subpopulations can be detected with the panels of present invention.

In a second aspect the present invention relates to a reagent composition for the flow cytometric immunophenotyping of blood cells comprising fluorochrome-conjugated antibodies directed against one of the following combination of markers:
(a) CD45, Ly6C, B220, CCR2, CD11b, CD11c, CD172a, CD49b, CD80, CD86, F4/80, Ly6G, MHC-II, Siglec-F; or
(b) CD45, CCR4, CCR6, CD138, CD19, CD25, CD3, CD4, CD44, CD62L, CD8, CXCR3, TNF-RII, NK1.1.

In one embodiment the reagent composition of present invention comprises fluorochrome-conjugated antibodies directed against both combinations of markers of antibodies (a) and (b).

The reagent compositions comprising a panel of 14 antibodies can be advantageously used in immunophenotyping applying any method known in the prior art to detect subpopulations of cells from blood cells, preferably peripheral blood cells, of a mouse. In a preferred embodiment the method of present invention as detailed above is used.

In one embodiment of the reagent composition of present invention each antibody in the combination of markers (a) and (b) is conjugated to one of the following fluorochromes VioBlue, VioGreen, BV570, BV605, BV650, FITC, PE, ECD/PE-Vio615, PerCP/PE-Cy5/PC5, PerCP-Vio700/PC5.5, PE-Cy7/PE-Vio700/PC7, APC, AF700/APC5, APC-Fire^{™} 750/APC7.

Most preferred is the use of one specific fluorochrome per antibody, however, in certain setups two antibodies can also contain the same fluorochrome. It is also envisaged that other fluorochromes known in the prior art can be used as deemed suitable. The important criteria for the selection of the set of fluorochromes to be used in one marker set of present invention is that the fluorochromes need to have different fluorescence profiles so that it is possible to distinguish the signal of each fluorochrome from the others in the panel.

A further advantage of the method and panels of present invention is that it allows obtaining absolute values for the different subpopulations. In contrast to that, most immunological results in preclinical research to date are only reported as percentages (29), due to the need for washing regardless of whether the samples are from PB or isolated organs. The use of absolute values as in present invention enhances the translational nature of the results since the clinical study of the leukocyte component to observe possible alterations or pathologies is essentially based on absolute values (30, 31).

In one embodiment of the method of present invention the step (v) of determining the cell type according to the data obtained in step (iv) comprises obtaining the total amount of cells of each subpopulation as an absolute value.

As is further explained in the examples section the immune panels defined in this study were validated using an acute model of LPS-mediated lung inflammation. The results show that the immunological panels of present invention are sensitive enough to detect changes in PB at 24 hours post-LPS induction. The immunological changes that were detected with the method of present invention are consistent with findings in the literature on PB, even in other mouse strains such as BLAB/c (29) or SWISS (24), demonstrating that the panels are functional and valid for FCM-based studies of circulating immune populations.

The two novel panels of present invention are sensitive enough to detect changes in PB immune populations, even in minority populations. With these panels and using a low volume of blood, a very comprehensive study of the murine immune system, utilizing both absolute values and their relative percentages can be performed.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

The following examples and figures are provided by way of illustration and are not intended to limit the scope of the present invention.

### EXAMPLES

### Peripheral Blood Flow Cytometry panels design

Fourteen specific antibodies were selected, each one for a specific immune marker (Table 1 and 2) in 3 different cytometry panel: myeloid panel, lymphoid panel and intracellular panel. This selection for each panel was done considering the clone, the 14 available fluorochromes with its spectral overlap and the antigenic density of the immune marker.

All antibodies were titrated to determine their concentration (Tables 1 and 2 show the optimal volume used for each antibody), achieving an optimal balance between signal level for positive populations and negative populations. Additionally, red blood cell (RBC) lysis reagents were titrated to optimize erythrocyte removal from the blood samples.

### Validation of the panels in a model of acute lung inflammation

12 females of 18.25±1.04g (Charles River) were used and divided into two groups, control (received 100µl of saline intra-tracheally) and lipopolysaccharide (LPS). The LPS group received 5mg/kg of LPS (Sigma-Aldrich Ref: L2880) in 100µl of saline by intra-tracheal instillation (17). Two blood samples were obtained per animal: basal (before LPS administration) and 24 hours post induction (p.i.). Clinical variables (dehydration, piloerection, lack of cleanliness, lesions, aggressiveness, passivity and repeated behaviors) of the animals were monitored daily until sacrifice (3 days) and scored from 0 (no severity) to 4 (high severity). A cumulative score >16 points, or a score of 4 in any of the previously described variables was established as the endpoint criterion. Before starting, animal handling was performed for 5 days to reduce the effect of stress. animals were housed at constant ambient temperature, with natural light cycle (12-h photoperiod) and were fed ad libitum with standard diet and water. The animals were acclimatized in the facilities for 1 week after arrival.

### Sample collection

Two cytometry panels were used, panels were named for the immune populations they study, myeloid and lymphoid panel (MP and LP) 100 µl of PB (50µL for each panel) were collected from the maxillary venous sinus with a sterile lancet and stored in 500 µl ethylenediaminetetraacetic acid (EDTA, 500 µl VACUTEST) tubes. Before collection, mice were anesthetized with 3% sevoflurane in 100% oxygen. After each extraction the animals received 100µL of saline intraperitoneally to replace the volume extracted.

### Sample processing

All antibodies were titrated using different concentrations in order to choose the one that results in the highest signal from the positive population along with the lowest signal from the negative population. In addition, lysis reagent was titrated in concentration timing to get the most optimal erythrocyte lysate from the blood. Samples were processed within 1 hour after the extraction. Sample data acquisition was carried out on a MACSQuant Analyser 16 cytometer (Miltenyi Biotec, Bergisch Gladbach, Germany).

For MP and LP fresh PB was labeled with a mixture of surface antibodies and incubated at room temperature. After surface labelling, red blood cells (RBC) were lysed (not washed) with RBC Lysis/Fixation Buffer (Biolegend, San Diego, CA, USA). Antibody labelling was performed according to table 1 and 2 which permit to visualize the main leukocytes populations.

### Data analysis

Cytometry data from both studies were analyzed by classical manual gating of known subpopulations (Table 3-4), using Kaluza software (Kaluza 2.1 version, Beckman Coulter, Brea, CA, USA). Data files were encrypted and the analyses were done blind. Immune populations were measured as cells per microliter (cls/µL).

### Statistical analysis

The statistical analysis was conducted using the BlueSky Statistic program. The threshold for statistical significance was set at p ≤ 0.05 for all analyses. Our data did not violate the assumptions of normality and homogeneity of variances. Subsequently, Student's t-tests were performed to analyze the differences between groups.

### Ethics

Animals were housed in the animal facility of the Hospital General Universitario Gregorio Marañón (HGUGM), Madrid, Spain (authorization. ES280790000087). All animal procedures conformed to the EU Directive 2010/63EU and national regulations (RD 53/2013). All animal procedures were approved by the HGUGM Animal Experimentation Ethics Committee and by the Animal Protection Board of the Comunidad Autónoma de Madrid (PROEX 335.6/21).

**Table 1: Antibodies used in the myeloid panel. The table shows the fluorophore and clone for each antibody, as well as the commercial brand and, reference. None of the antibodies are mouse strain-restricted.**

| **NAME** | **CHANNEL** | **COMERCIAL BRAND** | **Volumen in 50µL** | **REFERENCE** | **CLONE** |
|---|---|---|---|---|---|
| B220 | APC5 | Biolegend | 0.5µL | 103231 | RA3-6B2 |
| CCR2 | PC7 | Miltenyi | 1µL | 130-120-818 | REA538 |
| CD11b | PC5.5 | Miltenyi | 0.5µL | 130-113-809 | REA592 |
| CD11c | APC7 | Miltenyi | 1µL | 130-110-704 | REA754 |
| CD172a | VioBlue | Miltenyi | 0.5µL | 130-123-151 | REA1201 |
| CD45 | VioGreen | Miltenyi | 1µL | 130-123-900 | 30F11 |
| CD49b | ECD/PE-Vio615 | Miltenyi | 0.5µL | 130-116-437 | REA981 |
| Ly6C | PerCP/PC5 | Biolegend | 0.7µL | 128028 | HK1.4 |
| CD80 | APC | Miltenyi | 1µL | 130-116-461 | REA983 |
| CD86 | APC | Miltenyi | 2.5µL | 130-102-558 | PO3.3 |
| F4/80 | FITC | Miltenyi | 0.5µL | 130-117-509 | REA126 |
| Ly6G | BV605 | Biolegend | 0.7µL | 127639 | 1A8 |
| MHC-II | BV650 | Biolegend | 0.7µL | 107641 | M5/114.15. 2 |
| Siglec-F | PE | Miltenyi | 1µL | 130-112-174 | REA798 |

**Table 2: Antibodies used in the lymphoid panel. The table shows the fluorophore and clone for each antibody, as well as the commercial brand and, reference. None of the antibodies are mouse strain restricted.**

| **NAME** | **CHANNEL** | **COMERCIAL BRAND** | **Volumen in 50µL** | **REFERENCE** | **CLONE** |
|---|---|---|---|---|---|
| CCR4 | PE | Biolegend | 2.5µL | 131204 | 2G12 |
| CCR6 | BV605 | Biolegend | 2.5µL | 129819 | 29-2L1 7 |
| CD138 | PC7 | Miltenyi | 1µL | 130-102-318 | REA104 |
| CD19 | BV570 | Biolegend | 1µL | 115535 | 6D5 |
| CD25 | FITC | Miltenyi | 1µL | 130-120-088 | REA568 |
| CD3 | VioBlue | Miltenyi | 1µL | 130-118-849. | 17A2 |
| CD4 | BV650 | Biolegend | 1.25µL | 100545 | RM4-5 |
| CD44 | APC5 | Biolegend | 0.5µL | 103026 | IM7 |
| CD45 | VioGreen | Miltenyi | 1µL | 130-123-900. | 30F11 |
| CD62L | PerCP/PC5 | Biolegend | 0.7µL | 104410 | MEL-14 |
| CD8 | ECD/PE-Vio615 | Miltenyi | 1µL | 130-123-914 | REA601 |
| CXCR3 | APC7 | Biolegend | 0.7µL | 126540 | CXCR3-173 |
| NK1.1 | APC | Miltenyi | 1µL | 130-120-507 | REA1162 |
| TNF-RII | PC5.5 | Miltenyi | 2µL | 130-104-701 | REA228 |

**Table 3: Manual gating strategy by differential expression of extracellular markers for the myeloid panel.**

| **PB Population** | **Marker** |
|---|---|
| Leukocytes | CD45+ |
| Myeloid cells | CD45⁺ CD172a⁺ |
| Granulocytes | CD45⁺ CD172a+ CD11b⁺ |
| Basophils | CD45+ CD172a+ CD11b⁺ CD49b⁺ SiglecF⁻ CD11c⁻ |
| Neutrophils | CD45+ CD172a+CD11b⁺ MHCII⁻ Ly6G⁺ F4/80⁻ |
| Eosinophils | CD45+ CD172a+CD11b⁺ Ly6G⁻ F4/80⁺ SiglecF⁺ |
| Monocytes | CD45+ CD172a+CD11b⁺ MHCII⁻ F4/80⁺ Ly6G⁻ |
| Classical/Immature Monocytes | CD45+ CD172a+CD11b+ MHCII- F4/80+ Ly6G- Ly6C⁺ CCR2⁺CD49b⁺ CD11c⁻ |
| Non-Classical/ Mature Monocytes | CD45+ CD172a+CD11b+ MHCII- F4/80+ Ly6G- Ly6C⁻ CCR2⁻ CD49b⁻ CD11c⁺ |
| Induced Monocytes type I | CD45+ CD172a+ CD11b⁺ MHCII^{high} F4/80⁺ |
| Induced Monocytes type II | CD45+ CD172a+ CD11b⁺ MHCII^{low} F4/80⁺ |
| Dendritic cells (DCs) | CD45+ CD172a+ CD11c⁺ Ly6C⁺ |
| Conventional DCs | CD45+ CD172a+ CD11c+ Ly6C+ B220⁻ MHCII⁺ F4/80⁺ |
| Type I DC (Ininflamatory) | CD45+ CD172a+ CD11c+ Ly6C+ B220- MHCII⁺ F4/80+ CD11b⁺ |
| Mature DCs | CD45+ CD172a+ CD11c+ Ly6C+ B220- MHCII⁺ F4/80+ CD11b+ CD80⁺/CD86⁺ |
| Type II DCs | CD45+ CD172a+ CD11c+ Ly6C+ B220- MHCII⁺ F4/80+ CD11b⁻ |
| Plasmacytoid DCs | CD45+ CD172a+ CD11c+ Ly6C+ B220⁺ MHCII^{low}CD11b⁻ |
| Myeloid derived supresor cells (MDSC) | CD45+ CD172a+ CD11b⁺ CD11c⁻ F4/80^{low} MHCII⁻ |
| Monocytic-MDSC | CD45+ CD172a+CD11b+ CD11c- F4/80low MHCII- Ly6G^{- /low} Ly6C⁺ CCR2⁺ |
| Polymorfonuclear-MDSC | CD45+ CD172a+ CD11b+ CD11c- F4/80low MHCII-Ly6G+Ly6C^{-/low} CCR2⁻ |

**Table 4: Manual gating strategy by differential expression of extracellular markers for the lymphoid panel.**

| **PB Population** | **Marker** |
|---|---|
| T lymphocytes | CD45⁺ CD3⁺ |
| Activated T lymphocytes | CD45+ CD3⁺ CD25⁺ |
| Naive T lymphocytes | CD45+ CD3+ CD62L⁺CD44⁻ |
| Central memory T lymphocytes | CD45+ CD3+ CD62L⁺CD44⁺ |
| Effector T lymphocytes | CD45+ CD3+CD62L⁻CD44⁺ |
| CD4⁺T lymphocytes | CD45+ CD3+ CD4⁺ CD8⁻ |
| CD4+ T lymphocytes: Naive, Activated, central memory, effector | CD45⁺ CD3⁺ CD4⁺: CD62L⁺ CD44⁻ , CD25⁺, CD62L⁺ CD44⁺, CD44⁺ |
| Th1 lymphocytes | CD45+ CD3+ CD4+ CXCR3⁺CCR6⁻ CCR4⁻ |
| Th1 lymphocytes Naive, Activated, central memory, effector | Th1 CD62L⁺ CD44⁻ , CD25⁺, CD62L⁺ CD44⁺, CD44⁺ |
| Th2 lymphocytes | CD45+ CD3+CD4+ CCR6⁻CCR4⁺ |
| Th2 lymphocytes: Naive, Activated, central memory, effector | Th2 CD62L⁺ CD44⁻ , CD25⁺, CD62L⁺ CD44⁺, CD44⁺ |
| Th17 lymphocytes | CD45+ CD3+ CD4+ CCR6⁺CCR4⁺ NK1.1⁺ |
| Th17 lymphocytes: Naive, Activated, central memory, effector | Th17 CD62L⁺ CD44⁻ , CD25⁺, CD62L⁺ CD44⁺, CD44⁺ |
| Th9 lymphocytes | CD45+ CD3+ CD4+ CCR6⁺CCR4⁻ |
| Th9 lymphocytes Naive, Activated, central memory, effector | Th9: CD62L⁺ CD44⁻ , CD25⁺, CD62L⁺ CD44⁺, CD44⁺ |
| Treg lymphocytes | CD45+ CD3+ CD4+ TNFRII⁺CD25⁺ CTLA4⁺ |
| CD8⁺T lymphocytes | CD45+ CD3+ CD8⁺ CD4⁻ |
| CD8+ T lymphocytes: Naive, Activated, central memory, effector | CD45⁺ CD3⁺ CD8+ CD4⁻ CD62L⁺ CD44⁻ , CD25⁺, CD62L⁺ CD44⁺, CD44⁺ |
| Natural killers cells (NK) | CD45+ CD3⁻ NK1.1⁺ |
| NKT lymphocytes | CD45+ CD3⁺ NK1.1⁺ |
| B Lymphocytes | CD45+ CD19⁺ CD3⁻ |
| Plasma cells | CD45+ CD138⁺ CD3⁻ |

### Results

At 24 hours post-injection, absolute numbers of the neutrophil subset was untouched by the use of LPS compared to the control groups. However, myeloid cells are decreased in the LPS group compared to controls. Specifically, monocytic myeloid-derived suppressor cells (M-MDSCs) showed a pronounced decrease in the LPS group compared to controls (Fig 1B). Similarly, lymphoid lineage populations showed a significant reduction compared to the control group, with notable decreases in CD4+ T cells (and its effector and effector memory subsets), Th9 cells, CD8+ T cells (and its central memory and effector memory subset), B lymphocytes, plasma cells, natural killer (NK) and NK lymphocytes (NKT) (Fig 1). In summary, at 24 hours, intratracheal instillation of LPS induced a decrease in many populations compared to controls, except for neutrophils.

### Discussion

The cytometry panels developed in this study allow for extensive characterization of a wide range of immune subpopulations in mice, utilizing minimal peripheral blood volumes. This is especially useful for conducting longitudinal studies without adversely impacting the animal's immune physiology. The optimized panels of present invention address many of the limitations previously encountered in flow cytometry (FCM) studies of peripheral blood (PB) in rodents by employing very small blood samples. Similar methodologies for analyzing immune subpopulations with such small blood volumes have not been documented in the literature.

For instance, previous studies often extracted blood from the retro-orbital sinus, which required larger volumes, resulting in the animal's inability to recover (4). Additionally, most research involving PB cytometry is conducted at the endpoint, with the animal's entire circulating blood volume collected for various FCM panels or other techniques such as immunohistochemistry (19-21). The complexity of the immune response, involving multiple interrelated lineages, often results in FCM studies that focus on major immune populations, such as total lymphocytes (24), or specific immunological parameters like cytokines (25). Even studies focusing on isolated organs (such as lung (26), spleen (27), liver (28), etc.) often examine a limited number of immune subpopulations.

The 14-marker panels we designed allow us to study more than 50 immune subsets by carefully selecting the markers used. Previous studies that used fewer markers (20,22) or equipment with limited fluorescence channels (21) generally analyzed fewer populations. The limited commercial availability of conjugated antibodies to different fluorophores in mice complicates the design of complex panels. Additionally, many preclinical immunological studies report results as percentages due to the need for washing, limiting their ability to obtain absolute cell counts. Our approach, which uses small blood volumes without needing counting microbeads, allows obtaining absolute values for numerous subpopulations, aligning with the standard practice in clinical studies (30, 31).

The immune panels defined in this study were validated using an acute model of lipopolysaccharide (LPS)-mediated lung inflammation. This model was chosen for its biological relevance, cost-effectiveness, and reproducibility (24). Our results show that these immunological panels are sensitive enough to detect changes in PB at 24 hours post-LPS induction. The observed decrease in circulating immune populations in the LPS group may be due to cell migration to inflamed lung tissue, consistent with findings from other studies in different mouse strains (29). This suggests that our panels are valid for FCM-based studies of circulating immune populations in various experimental conditions.

### References

1. Fleisher, T. & Oliveira, J. 1239-1251.e1231 (2019).
2. Maecker, H.T., McCoy, J.P. & Nussenblatt, R. Standardizing immunophenotyping for the Human Immunology Project. Nature Reviews Immunology 12, 191-200 (2012).
3. Tessema, B., Boldt, A., König, B., Maier, M. & Sack, U. Flow-Cytometry Intracellular Detection and Quantification of HIV1 p24 Antigen and Immunocheckpoint Molecules in T Cells among HIV/AIDS Patients. HIV/AIDS (Auckland, N.Z.) 14, 365-379 (2022).
4. Gilmour, K.C. Flow Cytometry Confirmation Post Newborn Screening for SCID in England. International journal of neonatal screening 8 (2021).
5. Fang, H. et al. Acute promyelocytic leukemia: Immunophenotype and differential diagnosis by flow cytometry. Cytometry Part B: Clinical Cytometry 102, 283-291 (2022).
6. Hoggatt, J., Hoggatt, A.F., Tate, T.A., Fortman, J. & Pelus, L.M. Bleeding the laboratory mouse: Not all methods are equal. Experimental hematology 44, 132-137.e131 (2016).
7. Kiefer, J. et al. An Unbiased Flow Cytometry-Based Approach to Assess SubsetSpecificCirculating Monocyte Activation and Cytokine Profile in Whole Blood. Frontiers in immunology 12, 641224 (2021).
8. Stokes, J.V. et al. An optimized five-color/seven-parameter flow cytometry panel for immunophenotyping guinea pig peripheral blood lymphocytes. Journal of immunological methods 476, 112682 (2020).
9. Tarrant, J.M. The role of flow cytometry in companion animal diagnostic medicine. The Veterinary Journal 170, 278-288 (2005).
10. Weaver, J.L., Broud, D.D., McKinnon, K. & Germolec, D.R. SERIAL PHENOTYPIC ANALYSIS OF MOUSE PERIPHERAL BLOOD LEUKOCYTES. Toxicology Mechanisms and Methods 12, 95-118 (2002).
11. Wolforth, J. Methods of Blood Collection in the Mouse. Lab Animal 29, 47-53 (2000).
12. Rathkolb, B. et al. Blood Collection from Mice and Hematological Analyses on Mouse Blood. Current Protocols in Mouse Biology 3, 101-119 (2013).
13. Ma, C. et al. Activin A regulates activities of peripheral blood natural killer cells of mouse in an autocrine and paracrine manner. Experimental Cell Research 374, 114-121 (2019).
14. Shahneh, F. et al. Inflammatory Monocyte Counts Determine Venous Blood Clot Formation and Resolution. Arteriosclerosis, thrombosis, and vascular biology 42, 145-155 (2022).
15. Yu, Y.R. et al. A Protocol for the Comprehensive Flow Cytometric Analysis of Immune Cells in Normal and Inflamed Murine Non-Lymphoid Tissues. PloS one11, e0150606 (2016).
16. Del Zotto, G. et al. Comprehensive Phenotyping of Peripheral Blood T Lymphocytes in Healthy Mice. Cytometry Part A 99, 243-250 (2021)
17. Mertens, S. et al. Effect of three different forms of handling on the variation of aggression-associated parameters in individually and group-housed male C57BL/6NCrl mice. PloS one 14, e0215367 (2019).
18. Breznik, J.A., Schulz, C., Ma, J., Sloboda, D.M. & Bowdish, D.M.E. Biological sex, not reproductive cycle, influences peripheral blood immune cell prevalence in mice. The Journal of physiology 599, 2169-2195 (2021).
19. Xie, K. et al. Deep phenotyping and lifetime trajectories reveal limited effects of longevity regulators on the aging process in C57BL/6J mice. Nature Communications 13, 6830 (2022).
20. Chen, Z. et al. Radiation exposure lymphocyte damage assessed by γ-H2AX level using flow cytometry. Scientific Reports 14, 4339 (2024).
21. Idova, G.V., Al'perina, E.L., Gevorgyan, M.M., Tikhonova, M.A. & Zhanaeva, S.Y. Content of Peripheral Blood T- and B-Cell Subpopulations in Transgenic A53T Mice of Different Age (A Model of Parkinson's Disease). Bulletin of experimental biology and medicine 170, 401-404 (2021).
22. Zhou, J.R. et al. The role of memory T cells in Echinococcus granulosus-induced sensitization. Immun Inflamm Dis 11, e948 (2023).
23. Liverani, E. et al. LPS-induced systemic inflammation is more severe in P2Y12 null mice. Journal of leukocyte biology 95, 313-323 (2014).
24. Tewari, A., Bedi, J., Singh, B. & Gill, J.P.S. Oral exposure of deltamethrin and/or lipopolysaccharide (LPS) induced activation of the pulmonary immune system in Swiss albino mice. Environmental science and pollution research international25, 15436-15448 (2018).
25. Fonseca dos Reis, E., Viney, M. & Masuda, N. Network analysis of the immune state of mice. Scientific Reports 11, 4306 (2021).
26. Liu, J. et al. Mesenchymal stem cell-mediated immunomodulation of recruited mononuclear phagocytes during acute lung injury: a high-dimensional analysis study. Theranostics 11, 2232-2246 (2021).
27. Natalini, A. et al. OMIP-079: Cell cycle of CD4+ and CD8+ naïve/memory T cell subsets, and of Treg cells from mouse spleen. Cytometry Part A 99, 1171-1175 (2021).
28. Vanekova, L., Polidarova, M.P., Veverka, V., Birkus, G. & Brazdova, A. Multiparametric Flow Cytometry-Based Immunophenotyping of Mouse Liver Immune Cells. Methods and protocols 5, 70 (2022).
29. Zhang, Q. et al. Chrysosplenol D protects mice against LPS-induced acute lung injury by inhibiting oxidative stress, inflammation, and apoptosis via TLR4-MAPKs/NF-κB signaling pathways. 27, 514-524 (2021).
30. Yi, J.S. et al. Establishment of normative ranges of the healthy human immune system with comprehensive polychromatic flow cytometry profiling. PloS one 14, e0225512 (2019).
31. Baron, U. et al. Epigenetic immune cell counting in human blood samples for immunodiagnostics. Science Translational Medicine 10, eaan3508 (2018).

## Claims

1. A method for immunophenotyping of a mouse comprising the steps of:
(i) providing a blood sample of the mouse,
(ii) selecting a panel of fluorescently labeled antibodies,
(iii) incubating the selected panel of fluorescently labeled antibodies with the blood sample to be tested,
(iv) detecting the signal by flow cytometry to obtain detection data, and
(v) determining the cell type according to the data obtained in step (iv),
wherein the panel of fluorescently labeled antibodies comprises at least one of the following sets of markers:
(a) CD45, Ly6C, B220, CCR2, CD11b, CD11c, CD172a, CD49b, CD80/CD86, F4/80, Ly6G, MHC-II, Siglec-F, or
(b) CD45, CCR4, CCR6, CD138, CD19, CD25, CD3, CD4, CD44, CD62L, CD8, CXCR3, TNF-RII, NK1.1,
wherein the volume of the blood sample of the mouse provided in (i) is less than 100 µl per set of markers.

2. The method of claim 1, wherein the selected panel comprises both sets of markers (a) and (b).

3. The method of claim 1 or 2, wherein the blood sample of the mouse provided in step (i) is less than about 80 µl, preferably less than about 70 µl, most preferred less than about 60 µl per set of markers.

4. The method of claim 1 or 2, wherein the blood sample of the mouse provided in step (i) is between about 50 to 100 µl per set of markers, most preferred is an amount of between about 50 to 80 µl per set of markers.

5. The method of any of the preceding claims, wherein the blood sample is a peripheral blood sample.

6. The method of any one of the preceding claims, wherein the step (v) determining the cell type according to the data obtained in step (iv) comprises simultaneously detecting more than 30, more preferably more than 40, most preferred more than 50 subpopulations of cells.

7. The method of any one the preceding claims, wherein the step (v) determining the cell type according to the data obtained in step (iv) comprises obtaining the total amount of cells of each subpopulation as an absolute value.

8. A reagent composition for the flow cytometric immunophenotyping of blood cells comprising fluorochrome-conjugated antibodies directed against at least one of the following sets of markers:
(a) CD45, Ly6C, B220, CCR2, CD11b, CD11c, CD172a, CD49b, CD80, CD86, F4/80, Ly6G, MHC-II, Siglec-F, or
(b) CD45, CCR4, CCR6, CD138, CD19, CD25, CD3, CD4, CD44, CD62L, CD8, CXCR3, TNF-RII, NK1.1.

9. The reagent composition of claim 8 or the method of any one of claims 1 to 8, wherein each antibody in the sets of markers (a) and (b) is conjugated to one of the following fluorochromes VioBlue, VioGreen, BV570, BV605, BV650, FITC, PE, ECD/PE-Vio615, PerCP/PE-Cy5/PC5, PerCP-Vio700/PC5.5, PE-Cy7/PE-Vio700/PC7, APC, AF700/APC5, APC-Fire^{™} 750/APC7.

10. The reagent composition of claim 9 or the method of any one of claims 1 to 7, wherein each antibody in the sets of markers (a) and (b) is conjugated to a different fluorochrome.
